# EUROPEAN PATENT APPLICATION

(11) **EP 1 630 167 A1**
(43) Date of publication of application: **01.03.2006**
(21) Application number: 04291757.5
(22) Date of filing: 09.07.2004
(51) Int. Cl.: C07J 31/00, C07J 75/00

(54) **Polymorphs of 19-Nor steroid derivatives**

(71) Applicant: Proskelia SAS, 92230 Romainville (FR)
(72) Inventor: Nique, François, 94170 Le Perreux sur Marne (FR)
(74) Representative: Hirsch & Associés

(57) **Abstract**

Crystalline polymorphic Forms A or B of 11β-[4-[5-[(4,4,5,5,5-pentafluoropentyl)sulfonyl]pentyloxy] phenyl]-estra-1,3,5(10)-triene-3,17β-diol, their preparation, use and the pharmaceutical compositions comprising Form B.

## Description

The present invention relates to crystalline polymorphic forms of 19-nor steroid derivatives having a thiocarbonated chain in position 11β having a potent effect on breast cancer.

European patent EP 623140 describes 19-nor steroids having a thiocarbonated chain in position 11β and derivatives of the formula : 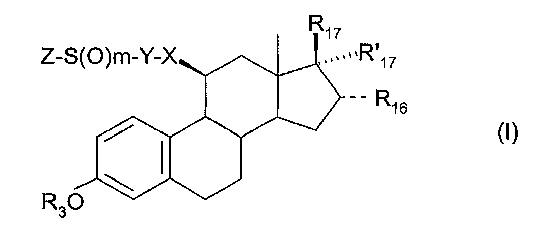 in which, among others
R₁₇ is a hydroxyl radical and R'₁₇ represents a hydrogen atom,
R₃ represents a hydrogen atom,
R₁₆ represents a hydrogen atom,
m = 1 or 2,
X represents an arylene group, having at most 10 carbon atoms linked to the steroid by a carbon atom,
Y represents a saturated or unsaturated, linear or branched aliphatic chain, containing 1 to 18 carbon atoms, optionally interrupted by an oxygen atom, and
Z represents a linear or branched alkyl radical, containing 1 to 8 carbon atoms and optionally substituted, by one or more radicals chosen from halogens, amino, alkylamino or dialkylamino, hydroxyl, among others.

The 11β-[4-[5-[(4,4,5,5,5-pentafluoropentyl)sulfonyl]pentyloxy]phenyl]-estra-1,3,5(10)-triene-3,17β-diol is described.

It has now been shown that 2 crystalline forms of the 11β-[4-[5-[(4,4,5,5,5-pentafluoropentyl)sulfonyl]pentyloxy] phenyl]-estra-1,3,5(10)-triene-3,17β-diol can be prepared and identified. These polymorphs have been named Form A and Form B.

One of these polymorphs designated as Form B has been found to have particularly potent properties and a particularly good stability.

Form A and Form B of the polymorphs can be defined by their X-ray powder diffraction patterns expressed in terms of "d" spacing, using Cu Kα radiation (λ = 1.54051 Å - room temperature) and the relative intensities percentages thereof.

### Form A

can be defined as follows (wave length : 1.54051 Å) - figure I :

| Peak position 2 Theta angle (°) | D space (Å) | relative intensities (%) | Counts number |
|---|---|---|---|
| 2.7950 | 15.7960 | 36.44 | 1346 |
| 3.9950 | 11.0559 | 17.92 | 662 |
| 4.7450 | 9.3115 | 22.39 | 827 |
| 5.1450 | 8.5893 | 20.90 | 772 |
| 5.5850 | 7.9145 | 24.01 | 887 |
| 5.7750 | 7.6549 | 24.45 | 903 |
| 7.1650 | 6.1755 | 56.23 | 2077 |
| 8.0450 | 5.5038 | 100.00 | 3694 |
| 8.5950 | 5.1540 | 68.38 | 2526 |
| 9.2950 | 4.7689 | 70.79 | 2615 |
| 9.6950 | 4.5739 | 63.16 | 2333 |
| 10.4250 | 4.2568 | 60.58 | 2238 |
| 11.4950 | 3.8651 | 37.79 | 1396 |
| 12.6150 | 3.5268 | 28.51 | 1053 |

White to slightly yellow white crystals. Melting point # 110°C.

### Form B

can be defined as follows (wave length : 1.54051 Å) - figure II :

| Peak position 2 Theta angle (°) | D space (Å) | relative intensities (%) | Counts number |
|---|---|---|---|
| 3.6890 | 11.9715 | 24.38 | 9978 |
| 3.9890 | 11.0725 | 42.16 | 17259 |
| 4.7590 | 9.2841 | 32.77 | 13414 |
| 5.5290 | 7.9944 | 33.35 | 13652 |
| 6.0190 | 7.3457 | 23.44 | 9596 |
| 6.7690 | 6.5350 | 39.87 | 16322 |
| 7.3290 | 6.0381 | 77.52 | 31731 |
| 7.3890 | 5.9893 | 71.04 | 29080 |
| 7.4890 | 5.9098 | 36.15 | 14798 |
| 7.7790 | 5.6907 | 19.80 | 8105 |
| 8.2590 | 5.3621 | 33.47 | 13701 |
| 8.4490 | 5.2424 | 78.45 | 32114 |
| 8.6190 | 5.1397 | 82.67 | 33841 |
| 9.0490 | 4.8974 | 75.75 | 31006 |
| 9.2590 | 4.7872 | 44.14 | 18069 |
| 9.5690 | 4.6335 | 48.89 | 20013 |
| 10.1490 | 4.3713 | 48.49 | 19850 |
| 10.3890 | 4.2714 | 100.00 | 40934 |
| 10.6890 | 4.1528 | 45.62 | 18673 |
| 10.8390 | 4.0960 | 37.95 | 15534 |
| 11.0490 | 4.0191 | 43.69 | 17883 |
| 11.3190 | 3.9244 | 28.13 | 11513 |
| 11.7590 | 3.7795 | 27.69 | 11335 |
| 12.0590 | 3.6869 | 13.87 | 5677 |
| 12.5590 | 3.5423 | 32.76 | 13412 |
| 13.3390 | 3.3386 | 27.82 | 11388 |
| 13.6190 | 3.2712 | 12.62 | 5166 |
| 13.8990 | 3.2066 | 12.34 | 5050 |
| 14.3490 | 3.1080 | 11.71 | 4793 |
| 15.7690 | 2.8343 | 12.47 | 5105 |
| 16.2090 | 2.7594 | 12.06 | 4935 |
| 16.9190 | 2.6467 | 13.03 | 5335 |
| 17.4090 | 2.5745 | 13.22 | 5412 |
| 18.4290 | 2.4365 | 11.61 | 4753 |
| 18.7690 | 2.3939 | 11.10 | 4542 |
| 21.5590 | 2.0962 | 10.97 | 4492 |

White crystals. Melting point: F = 158 to 164°C, more specifically 160 - 162°C.

Form B has a particularly good stability as determined by comparison to the amorphous form, with the measure of the total amount of impurities in stressed conditions until 14 days.

Another object of the instant invention is a process for the production of Form A and Form B.

Form A can be prepared by crystallisation from amorphous 11β-[4-[5-[(4,4,5,5,5-pentafluoropentyl)sulfonyl]pentyloxy] phenyl]-estra-1,3,5(10)-triene-3,17β-diol in an alcohol such as propanol (i.e. n-propanol, i.propanol) or butanol (i.e. n-butanol, i.butanol, 2-butanol) optionally with the addition of an ether such as isopropyl ether, methyl isobutyl ether, dipropylether, ethyl butyl ether, ethyl isobutyl ether.

According to the process, the amorphous form of 11β-[4-[5-[(4,4,5,5,5-pentafluoropentyl)sulfonyl]pentyloxy] phenyl]-estra-1,3,5(10)-triene-3,17β-diol is dissolved in a sufficient amount of solvent at a temperature up to the reflux temperature of the medium, the mixture is optionally filtered to get rid of undissolved material, the crystallisation can be carried out at room temperature or below (for instance at the temperature of an ice bath) optionally with the addition of an ether, then cooled at a temperature around 0°C. The crystals are collected by filtration, washed and optionally dried under vacum or atmospheric pressure.

The term "sufficient amount" is an amount sufficient to dissolve the amorphous form.

Form B can be obtained from Form A, by heating above the melting temperature of Form A. Mainly by heating at the reflux temperature of water or of an organic liquid chosen among those that do not dissolve such Form and which is chosen with a suitable boiling point.
The boiling point of the organic liquid must range between the melting points of both polymorphs Form A and Form B, roughly between 100 and 155°C. In these conditions, polymorph A melts in the medium and recrystallizes into the other polymorphic form : Form B. All kinds of organic liquids can be chosen, provided that they have the above mentioned characteristics. Water can be a suitable medium for this transformation. The process can be also conducted in a suitable organic liquid or mixtures thereof at a temperature of between 100 and 150°C. It may be advantageous to carry out the crystallisation process under inert atmosphere such as nitrogen atmosphere.

The term "suitable organic liquid " means an organic liquid that does not dissolve Form A, has a boiling point between the melting points of both polymorphs Form A and Form B and allows the formation of crystals of Form B. The suitable organic liquid can be chosen from organic liquids such as ethers [for instance butyl ether (b.p. = 141 °C), methyl heptyl ether (b.p. = 150°C)], or such as linear or branched aliphatic, cycloaliphatic, aromatic or araliphatic hydrocarbons [for instance toluene (b.p. = 111°C), xylenes (b.p. # 141°C), cycloheptane (b.p. = 118°C), cyclooctane (b.p. = 150°C), dimethylcyclohexanes (b.p. # 120°C), octane (b.p. = 126°C), isopropylcyclohexane (b.p. = 146°C)].

Form A or Form B can be recovered by isolating the crystals, for example by filtration or evaporation of the solvent or for Form B by filtration or evaporation of the organic liquid or water, or by any other method for removing the solvent from the crystals or the crystals from the solvent.

The amorphous 11β-[4-[5-[(4,4,5,5,5-pentafluoropentyl)sulfonyl]pentyloxy] phenyl]-estra-1,3,5(10)-triene-3,17β-diol is prepared according to EP 623,140 incorporated herein as reference.

The instant invention includes the use of Form A or Form B as a purification mean for the preparation of highly purified solutions of 11β-[4-[5-[(4,4,5,5,5-pentafluoropentyl)sulfonyl]pentyloxy] phenyl]-estra-1,3,5(10)-triene-3,17β-diol.

According to a prefered embodiment of the invention Form B can be micronized. Micronization is conducted according to the known methods. It is to be understood that micronized forms of Form B are included within the instant invention.

The instant invention includes also a pharmaceutical composition comprising a therapeutically effective amount of Form B alone or in combination with other suitable and pharmaceutically acceptable carrier. Examples of pharmaceutical pharmaceutically acceptable carriers are already described in EP 623,140. The pharmaceutical composition comprising Form B are of high interest for their potent effect on breast cancer.

The purified solutions prepared from Form A and/or Form B are also comprised within the definition of the pharmaceutical compositions of the invention, for the purpose of injectable solutions or drinkable solutions.

The effective amount of 11β-[4-[5-[(4,4,5,5,5-pentafluoropentyl)sulfonyl]pentyl-oxy] phenyl]-estra-1,3,5(10)-triene-3,17β-diol is usually comprised between 25 and 250 mg/kg by intramuscular or subcutaneous injection, preferably at a dose of between 50 to 250 mg/kg or between 50 and 250 mg/kg by oral route. Preferably the scheme of administration is an injection once a week, every week, or once a month, every month. It can also be every day by oral route.

### Examples

### Preparation of polymorph A :

### Example 1 :

Amorphous 11β-[4-[5-[4,4,5,5,5-pentafluoropentyl)sulfonyl]pentyloxy]phenyl]-estra-1,3,5(10)-triene-3,17β-diol (100 mg), prepared as described in the international patent application WO 93/13123, is dissolved in isopropanol (0.4 ml) at room temperature. Addition of isopropyl ether (0.8 ml) under stirring induces the crystallization. The mixture is cooled in an ice bath. The crystals (needles) are collected by filtration, washed with cooled isopropyl ether and dried at 50°C under vacuum (1 mbar). A white solid is obtained (92 mg ; yield 92 %). Melting point: from 90 to 100 °C (not sharp) [Kofler hot stage apparatus].

### Example 2 :

Amorphous 11β-[4-[5-[4,4,5,5,5-pentafluoropentyl)sulfonyl]pentyloxy]phenyl]-estra-1,3,5(10)-triene-3,17β-diol (3.3 g) is dissolved in isopropanol (8 ml) at room temperature. Isopropyl ether (15 ml) is added with stirring and the mixture is cooled in an ice bath. The crystals are collected by filtration, washed with cooled isopropyl ether and dried at 50°C under vacuum. A white solid is obtained (2.72 g ; yield 82 %). Melting point: from 90 to 100°C (not sharp) [Kofler hot stage apparatus].

### Example 3 :

In a similar manner, the compound can be crystallized from various alcohols. The table below describes the amount of crystals obtained starting from 500 mg of amorphous 11 β-[4-[5-[4,4,5,5,5-pentafluoropentyl)sulfonyl]pentyloxy]phenyl]-estra-1,3,5(10)-triene-3,17β-diol dissolved in 2 ml of the indicated solvents.

| Solvent | Weight of crystalline material collected | Crystallization yield | Melting point |
|---|---|---|---|
| n-propanol | 241 mg | 48 % | 95 -105 °C |
| Isopropanol | 348 mg | 69 % | 95 -105 °C |
| n-butanol | 332 mg | 66 % | 95 - 105 °C |
| Isobutanol | 408 mg | 80 % | 95 -105 °C |
| 2-butanol | 424 mg | 84 % | 95 -105 °C |

### Example 4 :

Amorphous 11 β-[4-[5-[4,4,5,5,5-pentafluoropentyl)sulfonyl]pentyloxy]phenyl]-estra-1,3,5(10)-triene-3,17β-diol (1 g) is dissolved in isobutanol (2.5 ml) at the reflux temperature. On cooling in an ice bath the crystallization occurs. The crystals are collected by filtration, washed with cooled isopropyl ether and dried at 50°C under vacuum (10⁻² mbar) in the presence of phosphorous pentoxide. A whitish solid is obtained (0.750 g ; yield 75 %). Melting point: around 110 °C.

NMR spectrum (in CDCl₃), δ (ppm) : 0.34 (s, 3 H, Me 18) ; 2.52 (d, 1 H) ; 2.82 to 2.90 (m, 2 H), 2.95 to 3.10 (m, 5 H, contains CH₂SO₂) 3.70 (t, 1 H, H 17) ; 3.88 (t, 2H, CH₂O), 3.95 (bs, 1 H, H 11) ; 6.41 (dd, 1 H, H 2) ; 6.57 to 6.68 (3 H, H 4 and Ar o to OCH₂) ; 6.82 (d, 1 H, H1) ; 6.95 (d, 2 H, Ar m to OCH₂).

### X ray diffraction spectra :

| θ ° | Distances Å | Relative intensity % | Counts number |
|---|---|---|---|
| 2.7950 | 15.7960 | 36.44 | 1346 |
| 3.9950 | 11.0559 | 17.92 | 662 |
| 4.7450 | 9.3115 | 22.39 | 827 |
| 5.1450 | 8.5893 | 20.90 | 772 |
| 5.5850 | 7.9145 | 24.01 | 887 |
| 5.7750 | 7.6549 | 24.45 | 903 |
| 7.1650 | 6.1755 | 56.23 | 2077 |
| 8.0450 | 5.5038 | 100.00 | 3694 |
| 8.5950 | 5.1540 | 68.38 | 2526 |
| 9.2950 | 4.7689 | 70.79 | 2615 |
| 9.6950 | 4.5739 | 63.16 | 2333 |
| 10.4250 | 4.2568 | 60.58 | 2238 |
| 11.4950 | 3.8651 | 37.79 | 1396 |
| 12.6150 | 3.5268 | 28.51 | 1053 |

Wavelenght : 1.54051 Å

### Example 5 :

Amorphous 11 β-[4-[5-[4,4,5,5,5-pentafluoropentyl)sulfonyl]pentyloxy]phenyl]-estra-1,3,5(10)-triene-3,17β-diol (86.5 g) is dissolved in isobutanol (200 ml) at 40°C. The hot solution is filtered. On cooling the filtrate an exothermic crystallization occurs. The crystals are collected by filtration, washed with cooled isopropyl ether and dried at 50°C under vacuum (10⁻² mbar) in the presence of phosphorous pentoxide. A slightly yellowish white solid is obtained (72.7 g ; yield 84 %). Melting point : 103 - 104 °C.

### Preparation of polymorph B :

### Example 6 :

Polymorph A crystallized from isobutanol (250 mg, m.p. 95-100 °C) is suspended in distilled water (2.5 ml) with efficient stirring under a nitrogen atmosphere. The mixture is heated in an oil bath at 140 °C. The compound immediately melts to yield a gummy solid. The reflux is maintained while stirring quickly for 30 min in order to break the solid obtained into a white suspension. The compound is isolated by filtration and dried under vacuum (10⁻² mbar) in the presence of phosphorous pentoxide to provide the polymorph B as white crystals (214 mg ; yield 89 %), melting point 158°C [Kofler hot stage apparatus].

### Example 7:

Polymorph A prepared as above (300 mg) is suspended in butyl ether (3 ml) with efficient stirring under a nitrogen atmosphere. It is then heated in an oil bath at 155°C. The compound immediately melts and sticks to the wall of the vessel then recrystallizes under the other polymorphic form : Form B. The reflux is maintained with stirring for 50 min in order to turn the agglomerated solid to a white suspension. The mixture is cooled to room temperature then the compound is collected by filtration, washed with isopropyl ether and dried under vacuum (10⁻² mbar) to give the polymorph B as white crystals (252 mg ; yield 84 %). Melting point: 159-160 °C ; [α]_{D} : - 28° (c = 1 % in CHCl₃).

### Example 8:

Polymorph A prepared from isobutanol (33.2 g) is suspended in butyl ether (170 ml) with efficient stirring under an argon atmosphere. It is then heated in an oil bath at 155°C. The compound immediately melts and recrystallizes under the other polymorphic form : Form B. The reflux is maintained with stirring for 30 min in order to turn the agglomerated solid to a white suspension. After cooling the mixture in an ice bath, the compound is collected by filtration, washed with isopropyl ether, then dried at 80°C under vacuum (10⁻² mbar) in the presence of phosphorous pentoxide to give the polymorph B as white crystals (31 g ; yield 93 %). Melting point : 161°C ; [α]_{D} : - 28° (c = 1 % in CHCl₃).

### UV spectrum

(in ethanol) : max 231 nm (ε = 16800) ; max 282 nm (ε = 3850) ; max 288 nm (ε = 3800) ; inflexion 273 nm.
(in ethanol + 0.1 N NaOH) : max 231 nm (ε = 16900) ; max 282 nm (ε = 3200) ; max: 288 nm (ε = 3350) ; max 301 nm (ε = 3000) ; inflexion 247 nm.

### Example 9 :

Form A (61.45 g) is suspended in butyl ether (300 ml) with efficient stirring under an nitrogen atmosphere. It is then heated in an oil bath at 155°C. The compound immediately melts and recrystallizes under the other polymorphic form : Form B. The reflux is maintained with stirring for 30 min in order to turn the agglomerated solid to a white suspension. After cooling the mixture in an ice bath, the compound is collected by filtration, washed with isopropyl ether, then dried at 80 °C under vacuum in the presence of phosphorous pentoxide to give the Form B as white crystals (57.16 g ; yield 93 %). Melting point: 164°C.

NMR spectrum (in CDCl₃), δ (ppm) : 0.36 (s, 3 H, Me 18) ; 2.53 (d, 1 H) ; 2.82 to 2.90 (m, 2 H); 2.95 to 3.10 (m, 5 H, contains CH₂SO₂) ; 3.71 (t, 1 H, H 17); 3.89 (t, 3 H, CH₂O) ; 3.95 (t, 1 H, H 11) ; 6.42 (dd, 1 H, H 2) ; ≈ 6.60 (1 H, H 4) ; 6.62 and 6.97 (2 d, 4 H, OPh) ; 6.83 (d, 1 H, H1).

### X ray diffraction spectra :

| θ ° | Distances Å | Relative intensity % | Counts number |
|---|---|---|---|
| 3.6890 | 11.9715 | 24.38 | 9978 |
| 3.9890 | 11.0725 | 42.16 | 17259 |
| 4.7590 | 9.2841 | 32.77 | 13414 |
| 5.5290 | 7.9944 | 33.35 | 13652 |
| 6.0190 | 7.3457 | 23.44 | 9596 |
| 6.7690 | 6.5350 | 39.87 | 16322 |
| 7.3290 | 6.0381 | 77.52 | 31731 |
| 7.3890 | 5.9893 | 71.04 | 29080 |
| 7.4890 | 5.9098 | 36.15 | 14798 |
| 7.7790 | 5.6907 | 19.80 | 8105 |
| 8.2590 | 5.3621 | 33.47 | 13701 |
| 8.4490 | 5.2424 | 78.45 | 32114 |
| 8.6190 | 5.1397 | 82.67 | 33841 |
| 9.0490 | 4.8974 | 75.75 | 31006 |
| 9.2590 | 4.7872 | 44.14 | 18069 |
| 9.5690 | 4.6335 | 48.89 | 20013 |
| 10.1490 | 4.3713 | 48.49 | 19850 |
| 10.3890 | 4.2714 | 100.00 | 40934 |
| 10.6890 | 4.1528 | 45.62 | 18673 |
| 10.8390 | 4.0960 | 37.95 | 15534 |
| 11.0490 | 4.0191 | 43.69 | 17883 |
| 11.3190 | 3.9244 | 28.13 | 11513 |
| 11.7590 | 3.7795 | 27.69 | 11335 |
| 12.0590 | 3.6869 | 13.87 | 5677 |
| 12.5590 | 3.5423 | 32.76 | 13412 |
| 13.3390 | 3.3386 | 27.82 | 11388 |
| 13.6190 | 3.2712 | 12.62 | 5166 |
| 13.8990 | 3.2066 | 12.34 | 5050 |
| 14.3490 | 3.1080 | 11.71 | 4793 |
| 15.7690 | 2.8343 | 12.47 | 5105 |
| 16.2090 | 2.7594 | 12.06 | 4935 |
| 16.9190 | 2.6467 | 13.03 | 5335 |
| 17.4090 | 2.5745 | 13.22 | 5412 |
| 18.4290 | 2.4365 | 11.61 | 4753 |
| 18.7690 | 2.3939 | 11.10 | 4542 |
| 21.5590 | 2.0962 | 10.97 | 4492 |

Wavelenght : 1.54051 Å

### Example 10 :

Several batches of polymorph B prepared as above (151.69 g, m.p. 160 to 161 °C) are mixed and stirred in isopropyl ether (600 ml) for 1 hour at room temperature. The mixture is filtered and the solid is washed 3 times with isopropyl ether (50 ml).

The remaining colorless crystals are dried at 30-35 °C under vacuum to give 150 g of compound which is micronized to produce 137.2 g of white solid, melting point : 160.1 °C (capillary tube).

### Example 11 :

### Stability data in stressed conditions (vessel open to the atmosphere)

### Total amount of impurities determined by HPLC (%)

| | Temperature | Initial status | 24 hours | 7 days | 14 days |
|---|---|---|---|---|---|
| Amorphous Batch 8 | 30 °C | 2.6 | | 3.3 | 4.5 |
| | 40 °C | | | 4.2 | 6.2 |
| | 50°C | | | 6 | 11 |
| | 80 °C | | 24.4 | | |
| Amorphous Batch 7 | 80 °C | 2.6 | 29.8 | | |
| Amorphous Batch 6 | 80 °C | 1.2 | 33.6 | | |
| Amorphous Batch 10 | 50 °C | 2.4 | | 5.4 | 10 |
| Polymorph B Example 10 | | 0.5 | | 0.7 | 1.1 |
| Polymorph B Example 7 | | ≤1 | | ~ 1 | ~ 1 |

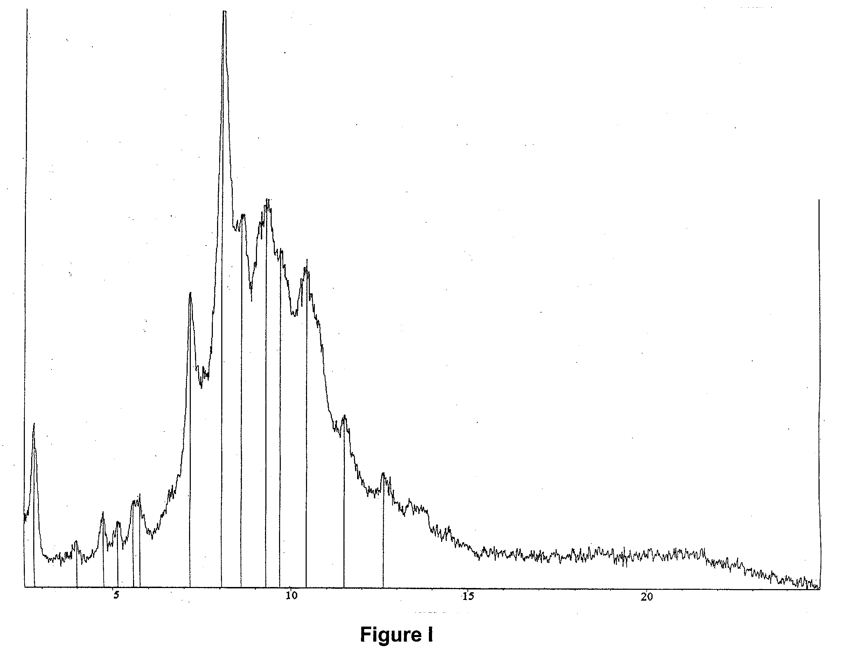

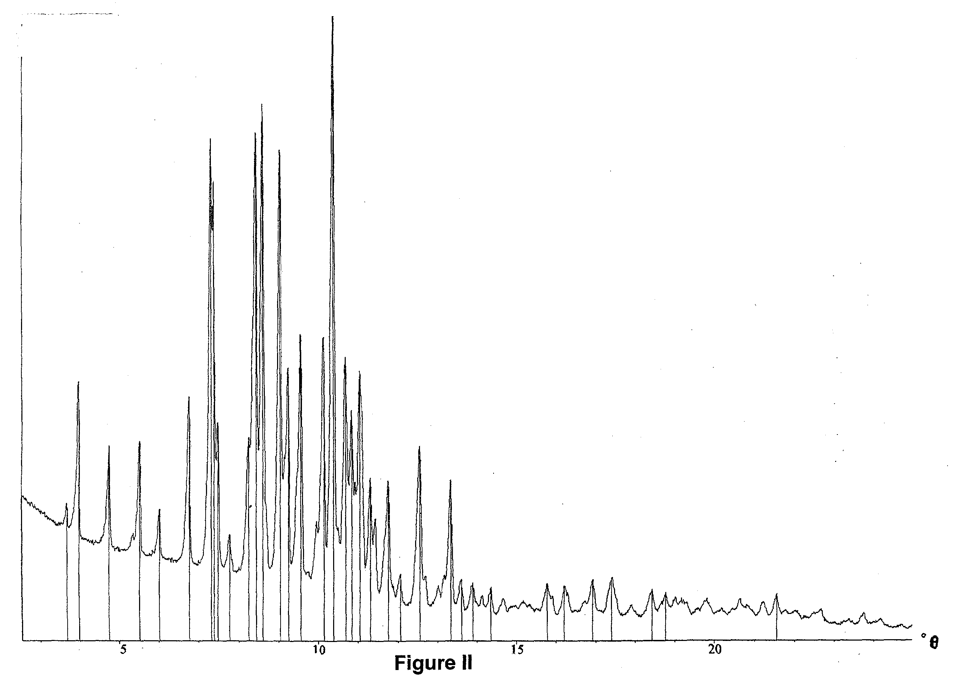

## Claims

1. Crystalline polymorphic Form B of 11β-[4-[5-[(4,4,5,5,5-pentafluoropentyl)sulfonyl]pentyloxy] phenyl]-estra-1,3,5(10)-triene-3,17β-diol in the form of white crystals, having an X-ray powder diffraction pattern expressed in terms of "d" spacing, using Cu Kα radiation (λ = 1.54051 Å - room temperature) and relative intensities percentages as given below :
| Peak position 2 Theta angle (°) | D space (Å) | relative intensities (%) | Counts number |
|---|---|---|---|
| 3.6890 | 11.9715 | 24.38 | 9978 |
| 3.9890 | 11.0725 | 42.16 | 17259 |
| 4.7590 | 9.2841 | 32.77 | 13414 |
| 5.5290 | 7.9944 | 33.35 | 13652 |
| 6.0190 | 7.3457 | 23.44 | 9596 |
| 6.7690 | 6.5350 | 39.87 | 16322 |
| 7.3290 | 6.0381 | 77.52 | 31731 |
| 7.3890 | 5.9893 | 71.04 | 29080 |
| 7.4890 | 5.9098 | 36.15 | 14798 |
| 7.7790 | 5.6907 | 19.80 | 8105 |
| 8.2590 | 5.3621 | 33.47 | 13701 |
| 8.4490 | 5.2424 | 78.45 | 32114 |
| 8.6190 | 5.1397 | 82.67 | 33841 |
| 9.0490 | 4.8974 | 75.75 | 31006 |
| 9.2590 | 4.7872 | 44.14 | 18069 |
| 9.5690 | 4.6335 | 48.89 | 20013 |
| 10.1490 | 4.3713 | 48.49 | 19850 |
| 10.3890 | 4.2714 | 100.00 | 40934 |
| 10.6890 | 4.1528 | 45.62 | 18673 |
| 10.8390 | 4.0960 | 37.95 | 15534 |
| 11.0490 | 4.0191 | 43.69 | 17883 |
| 11.3190 | 3.9244 | 28.13 | 11513 |
| 11.7590 | 3.7795 | 27.69 | 11335 |
| 12.0590 | 3.6869 | 13.87 | 5677 |
| 12.5590 | 3.5423 | 32.76 | 13412 |
| 13.3390 | 3.3386 | 27.82 | 11388 |
| 13.6190 | 3.2712 | 12.62 | 5166 |
| 13.8990 | 3.2066 | 12.34 | 5050 |
| 14.3490 | 3.1080 | 11.71 | 4793 |
| 15.7690 | 2.8343 | 12.47 | 5105 |
| 16.2090 | 2.7594 | 12.06 | 4935 |
| 16.9190 | 2.6467 | 13.03 | 5335 |
| 17.4090 | 2.5745 | 13.22 | 5412 |
| 18.4290 | 2.4365 | 11.61 | 4753 |
| 18.7690 | 2.3939 | 11.10 | 4542 |
| 21.5590 | 2.0962 | 10.97 | 4492 |

2. Crystalline polymorphic Form B of 11β-[4-[5-[(4,4,5,5,5-pentafluoropentyl)sulfonyl]pentyloxy] phenyl]-estra-1,3,5(10)-triene-3,17β-diol according to claim 1 having a melting point from 158 to 164°C.

3. Crystalline polymorphic Form B of 11β-[4-[5-[(4,4,5,5,5-pentafluoropentyl)sulfonyl]pentyloxy] phenyl]-estra-1,3,5(10)-triene-3,17β-diol according to claim 1 or 2, wherein the form is micronized.

4. A pharmaceutical composition comprising a therapeutically effective amount of Form B as defined in any of claim 1 or 2, alone or in combination with other suitable and pharmaceutically acceptable carrier.

5. Crystalline polymorphic Form A of 11 β-[4-[5-[(4,4,5,5,5-pentafluoropentyl)sulfonyl]pentyloxy] phenyl]-estra-1,3,5(10)-triene-3,17β-diol, having an X-ray powder diffraction pattern expressed in terms of "d" spacing, using Cu Kα radiation (λ = 1.54051 Å - room temperature) and relative intensities percentages as given below :
| Peak position 2 Theta angle (°) | D space (Å) | relative intensities (%) | Counts number |
|---|---|---|---|
| 2.7950 | 15.7960 | 36.44 | 1346 |
| 3.9950 | 11.0559 | 17.92 | 662 |
| 4.7450 | 9.3115 | 22.39 | 827 |
| 5.1450 | 8.5893 | 20.90 | 772 |
| 5.5850 | 7.9145 | 24.01 | 887 |
| 5.7750 | 7.6549 | 24.45 | 903 |
| 7.1650 | 6.1755 | 56.23 | 2077 |
| 8.0450 | 5.5038 | 100.00 | 3694 |
| 8.5950 | 5.1540 | 68.38 | 2526 |
| 9.2950 | 4.7689 | 70.79 | 2615 |
| 9.6950 | 4.5739 | 63.16 | 2333 |
| 10.4250 | 4.2568 | 60.58 | 2238 |
| 11.4950 | 3.8651 | 37.79 | 1396 |
| 12.6150 | 3.5268 | 28.51 | 1053 |

6. Crystalline polymorphic Form A of 11 β-[4-[5-[(4,4,5,5,5-pentafluoropentyl)sulfonyl]pentyloxy] phenyl]-estra-1,3,5(10)-triene-3,17β-diol according to claim 4 having a melting point of about 110°C.

7. Process for the preparation of Form B of 11 β-[4-[5-[(4,4,5,5,5-pentafluoropen-tyl)sulfonyl]pentyloxy] phenyl]-estra-1,3,5(10)-triene-3,17β-diol as defined in any of claims 1 or 2, comprising heating Form A, as defined in any of claims 4 or 5, above the melting temperature of Form A.

8. Process according to claim 6, for the preparation of Form B comprising heating Form A at the reflux temperature of water or an organic liquid chosen among those that does not dissolve such Form and which is chosen with a suitable boiling point.

9. Process for the preparation of Form A of 11 β-[4-[5-[(4,4,5,5,5-pentafluoropen-tyl)sulfonyl]pentyloxy] phenyl]-estra-1,3,5(10)-triene-3,17β-diol as defined in any of claims 3 or 4, comprising crystallisation from amorphous 11 β-[4-[5-[(4,4,5,5,5-pentafluoropentyl)sulfonyl]pentyloxy] phenyl]-estra-1,3,5(10)-triene-3,17β-diol in an alcohol, optionally with the addition of an ether.

10. Process according to claim 8, for the preparation of Form A, wherein the alcohol is n-propanol, i.propanol or n-butanol, i.butanol or 2-butanol.

11. Use of Form A or Form B of 11β-[4-[5-[(4,4,5,5,5-pentafluoropentyl)sulfonyl]pentyloxy] phenyt]-estra-1,3,5(10)-triene-3,17β-diol as defined respectively in claims 1 or 2, or 3 or 4, as a purification mean for the preparation of highly purified solutions of 11 β-[4-[5-[(4,4,5,5,5-pentafluoropentyl)sulfonyl]pentyl-oxy] phenyl]-estra-1,3,5(10)-triene-3,17β-diol.
